# EUROPEAN PATENT APPLICATION

(11) **EP 2 436 768 A1**
(43) Date of publication of application: **04.04.2012**
(21) Application number: 10182335.9
(22) Date of filing: 29.09.2010
(51) Int. Cl.: C12N 15/82

(54) **Method for inducing mutations and/or epimutations in plants.**

(71) Applicant: Institut National De La Recherche Agronomique, 75007 Paris (FR)
(72) Inventor: Vaucheret, Hervé, 75005 Paris (FR); Mallory, Allison, 75005 Paris (FR); Lepere, Gersende, 92100 Boulogne Billancourt (FR)
(74) Representative: Vialle-Presles, Marie José

(57) **Abstract**

The invention relates to a method for inducing mutations and/or epimutations in plants by transforming said plants with a DNA construct expressing a RNAse III protein.

SEQUENCE LISTING

<110> INSTITUT NATIONAL DE LA RECHERCHE AGRONOMIQUE
VAUCHERET, Hervé
MALLORY, Allison
LEPERE, Gersende

<120> Method for inducing mutations and/or epimutations in plants.

<130> MJP11-F539-149EP

<160> 23

<170> PatentIn version 3.5

<210> 1
<211> 391
<212> PRT
<213> Arabidopsis thaliana

<400> 1

Met Asp His Ser Ile Ser Pro Glu Tyr Asn Phe Pro Ala Ile Thr Arg
1               5                   10                  15

Cys Ser Leu Ser Asn Ser Leu Pro His Arg Pro Pro Ser Pro Leu Pro
            20                  25                  30

Ser Ser Ala Asp Ile His Arg Phe Tyr Asn Ser Leu Ser Pro Ser Ala
        35                  40                  45

Pro Ser Val Pro Val Ser Ser Glu Met Glu Ser Met Glu Ala Val Glu
    50                  55                  60

Lys Ile Leu Asn Tyr Lys Phe Ser Asn Lys Ser Leu Leu Lys Glu Ala
65                  70                  75                  80

Ile Thr His Thr Ser Cys Thr Asp Phe Pro Ser Tyr Glu Arg Leu Glu
                85                  90                  95

Phe Ile Gly Asp Ser Ala Ile Gly Leu Ala Ile Ser Asn Tyr Leu Tyr
            100                 105                 110

Leu Thr Tyr Pro Ser Leu Glu Pro His Asp Leu Ser Leu Leu Arg Ala
        115                 120                 125

Ala Asn Val Ser Thr Glu Lys Leu Ala Arg Val Ser Leu Asn His Gly
    130                 135                 140

Leu Tyr Ser Phe Leu Arg Arg Asn Ala Pro Ser Leu Asp Glu Lys Val
145                 150                 155                 160

Lys Glu Phe Ser Glu Ala Val Gly Lys Glu Asp Asp Leu Ser Val Ser
                165                 170                 175

Tyr Gly Gly Leu Val Lys Ala Pro Lys Val Leu Ala Asp Leu Phe Glu
            180                 185                 190

Ser Leu Ala Gly Ala Val Tyr Val Asp Val Asn Phe Asp Leu Gln Arg
        195                 200                 205

Leu Trp Val Ile Phe Arg Gly Leu Leu Glu Pro Ile Val Thr Leu Asp
    210                 215                 220

Asp Leu Gln Lys Gln Pro Gln Pro Val Ser Met Leu Phe Lys Leu Cys
225                 230                 235                 240

His Lys His Lys Lys Arg Ile Asp Ile Lys Asn Trp Lys Asp Gly Asn
                245                 250                 255

Val Ser Ile Ala Val Ile Tyr Leu Asp Asp Glu Leu Leu Ala Ser Gly
            260                 265                 270

Arg Ala Glu Asn Lys Asp Ile Ala Arg Leu Ile Ala Ala Lys Glu Ala
        275                 280                 285

Leu Arg Lys Leu Ser Glu Val Phe Pro Val Glu Met Val Ile Asp Glu
    290                 295                 300

Asp Ser Val Glu Ile Gln Leu Thr His Ala Lys Thr Lys Leu Asn Glu
305                 310                 315                 320

Ile Cys Leu Lys Lys Lys Trp Pro Lys Pro Ile Tyr Ser Val Glu Glu
                325                 330                 335

Asp Arg Ser Ser Val Gln Gly Lys Arg Phe Val Cys Ser Ala Lys Ile
            340                 345                 350

Lys Ile Thr Glu Glu Lys Thr Leu Tyr Met Lys Gly Asp Glu Gln Ser
        355                 360                 365

Lys Ile Lys Lys Ala Glu Ser Ser Ser Ala Tyr His Met Ile Arg Ala
    370                 375                 380

Leu Arg Lys Ser His Tyr Leu
385                 390

<210> 2
<211> 134
<212> PRT
<213> Arabidopsis thaliana

<400> 2

Leu Lys Glu Ala Ile Thr His Thr Ser Cys Thr Asp Phe Pro Ser Tyr
1               5                   10                  15

Glu Arg Leu Glu Phe Ile Gly Asp Ser Ala Ile Gly Leu Ala Ile Ser
            20                  25                  30

Asn Tyr Leu Tyr Leu Thr Tyr Pro Ser Leu Glu Pro His Asp Leu Ser
        35                  40                  45

Leu Leu Arg Ala Ala Asn Val Ser Thr Glu Lys Leu Ala Arg Val Ser
    50                  55                  60

Leu Asn His Gly Leu Tyr Ser Phe Leu Arg Arg Asn Ala Pro Ser Leu
65                  70                  75                  80

Asp Glu Lys Val Lys Glu Phe Ser Glu Ala Val Gly Lys Glu Asp Asp
                85                  90                  95

Leu Ser Val Ser Tyr Gly Gly Leu Val Lys Ala Pro Lys Val Leu Ala
            100                 105                 110

Asp Leu Phe Glu Ser Leu Ala Gly Ala Val Tyr Val Asp Val Asn Phe
        115                 120                 125

Asp Leu Gln Arg Leu Trp
    130

<210>  3
<211>  181
<212>  PRT
<213>  Arabidopsis thaliana

<400>  3

Val Ile Phe Arg Gly Leu Leu Glu Pro Ile Val Thr Leu Asp Asp Leu
1               5                   10                  15

Gln Lys Gln Pro Gln Pro Val Ser Met Leu Phe Lys Leu Cys His Lys
            20                  25                  30

His Lys Lys Arg Ile Asp Ile Lys Asn Trp Lys Asp Gly Asn Val Ser
        35                  40                  45

Ile Ala Val Ile Tyr Leu Asp Asp Glu Leu Leu Ala Ser Gly Arg Ala
    50                  55                  60

Glu Asn Lys Asp Ile Ala Arg Leu Ile Ala Ala Lys Glu Ala Leu Arg
65                  70                  75                  80

Lys Leu Ser Glu Val Phe Pro Val Glu Met Val Ile Asp Glu Asp Ser
                85                  90                  95

Val Glu Ile Gln Leu Thr His Ala Lys Thr Lys Leu Asn Glu Ile Cys
            100                 105                 110

Leu Lys Lys Lys Trp Pro Lys Pro Ile Tyr Ser Val Glu Glu Asp Arg
        115                 120                 125

Ser Ser Val Gln Gly Lys Arg Phe Val Cys Ser Ala Lys Ile Lys Ile
    130                 135                 140

Thr Glu Glu Lys Thr Leu Tyr Met Lys Gly Asp Glu Gln Ser Lys Ile
145                 150                 155                 160

Lys Lys Ala Glu Ser Ser Ser Ala Tyr His Met Ile Arg Ala Leu Arg
                165                 170                 175

Lys Ser His Tyr Leu
            180

<210>  4
<211>  29
<212>  DNA
<213>  Artificial

<220>
<223>  PCR primer

<400>  4
cgacctgcag atgggatcgc aactctcaa                                          29

<210>  5
<211>  30
<212>  DNA
<213>  Artificial

<220>
<223>  PCR primer

<400>  5
cggcgaattc ctaaaggtct ccgaaaaatt                                         30

<210>  6
<211>  30
<212>  DNA
<213>  Artificial

<220>
<223>  PCR primer

<400>  6
cgcgggatcc ctctctacca tggatcactc                                         30

<210>  7
<211>  30
<212>  DNA
<213>  Artificial

<220>
<223>  PCR primer

<400>  7
cgcggaattc ctgaactctg tccataacgg                                         30

<210>  8
<211>  31
<212>  DNA
<213>  Artificial

<220>
<223>  PCR primer

<400>  8
cggctgcaga tgaattcagt agaagcagta g                                    31


<210>  9
<211>  33
<212>  DNA
<213>  Artificial

<220>
<223>  PCR primer

<400>  9
cggcggatcc tcatagtctt ctcctcctcc ttt                                  33


<210>  10
<211>  32
<212>  DNA
<213>  Artificial

<220>
<223>  PCR primer

<400>  10
cggcggatcc atggcgactc ttcgtttcac tc                                   32


<210>  11
<211>  33
<212>  DNA
<213>  Artificial

<220>
<223>  PCR primer

<400>  11
cggcgaattc ttacaacata gagactagtc ttc                                  33


<210>  12
<211>  33
<212>  DNA
<213>  Artificial

<220>
<223>  PCR primer

<400>  12
cggcggtcga catggagctt tgttcttcat ctc                                  33


<210>  13
<211>  32
<212>  DNA
<213>  Artificial

<220>
<223>  PCR primer

<400>  13
cggcggaatt ctcagacagc tttaggttgg at                                   32

<210>  14
<211>  40
<212>  DNA
<213>  Artificial

<220>
<223>  PCR primer

<400>  14
gaatcgtacg cactcctaga acttttagga gcttcgatcc                                40


<210>  15
<211>  40
<212>  DNA
<213>  Artificial

<220>
<223>  PCR primer

<400>  15
ggatcgaagc tcctaaaagt tctaggagtg cgtacgattc                                40


<210>  16
<211>  44
<212>  DNA
<213>  Artificial

<220>
<223>  PCR primer

<400>  16
ccttcttacg cgcggctaga gttcataggc gccagtgcta ttgg                           44


<210>  17
<211>  44
<212>  DNA
<213>  Artificial

<220>
<223>  PCR primer

<400>  17
ccaatagcac tggcgcctat gaactctagc cgcgcgtaag aagg                           44


<210>  18
<211>  46
<212>  DNA
<213>  Artificial

<220>
<223>  PCR primer

<400>  18
cctttattcg cccggcttga gttcttcggc gcctctattc ttgagg                         46


<210>  19
<211>  46
<212>  DNA
<213>  Artificial

<220>
<223>  PCR primer

<400>  19
cctcaagaat agaggcgccg aagaactcaa gccgggcgaa taaagg                    46


<210>  20
<211>  43
<212>  DNA
<213>  Artificial

<220>
<223>  PCR primer

<400>  20
gagaacaacg cagccttgag tatctttgga gcccatatca ttg                       43


<210>  21
<211>  43
<212>  DNA
<213>  Artificial

<220>
<223>  PCR primer

<400>  21
caatgatatg ggctccaaag atactcaagg ctgcgttgtt ctc                       43


<210>  22
<211>  45
<212>  DNA
<213>  Artificial

<220>
<223>  PCR primer

<400>  22
ggttttgttt tgcgcggctg gaatatgtag gagcgaagat acagg                     45


<210>  23
<211>  45
<212>  DNA
<213>  Artificial

<220>
<223>  PCR primer

<400>  23
cctgtatctt cgctcctaca tattccagcc gcgcaaaaca aaacc                     45

## Description

The present invention relates to methods and tools for inducing mutations and/or epimutations in plants.

Mutations and epimutations can result in heritable phenotypic variation, and can arise spontaneously or in response to environmental stress. Unlike classical nucleic acid mutations, epimutations are heritable changes in gene expression not entailing a modification of the DNA sequence. Epimutations occur at a higher frequency than nucleic acid mutations because they are not associated to changes in DNA sequence but rely on changes (gain or loss) of epigenetic marks such as DNA methylation or histone modification. DNA mutations are rare because of high fidelity DNA replication and efficient DNA repair pathways. In contrast, subtle and/or transient relaxation of the epigenetic machinery can create or erase a diversity of epigenetic marks, and this state subsequently can be maintained through mitosis and/or meiosis. However, epimutations also revert to wild-type at higher frequency than mutations.

In both plants and animals, cytosine is primarily methylated in CpG dinucleotide sequences; however, in plants two other DNA methylation contexts exist: CpNpG and the less abundant asymmetric CpNpN (where N is A, C or T). It is now clear, at least for the Arabidopsis genome, that DNA methylation patterns are generated by the concerted actions of DNA methyltransferases and demethylases. The Arabidopsis METHYLTRANSFERASE1 (MET1) mainly maintains CpG methylation by adding one methyl group on newly synthetized DNA. CHRONOMETHYLASE 3 (CMT3) is responsible for majority of CpNpG methylation and a small amount of CpNpN methylation whereas DOMAINS REARRANGED METHYLTRANSFERASE 2 (DRM2) is involved in *de novo* methylation in all contexts and in the maintenance of CpNpN methylation. DRM2 is instructed by 24-nucleotide siRNAs that guide DNA methylation at homologous loci. DNA demethylation is carried out by DNA glycosylases of the DEMETER (DME) family, which has four members: DME, DEMETER-LIKE2 and 3 (DML2, DML3) and REPRESSOR OF SILENCING 1 (ROS1). The expression of DME is restricted to the reproductive central cell whereas ROS1, DML2 and DML3 are expressed in all plant organs. By using genome tiling arrays and dml mutants, (PENTERMAN et al., Proc Natl Acad Sci U S A, 104, 6752-7, 2007) have mapped 179 loci actively demethylated by DML enzymes scattered throughout the genome. Most of the regions modified by DML proteins contain transposons or repeated sequences and are located near to or overlap with protein-coding genes. Thus, it has been proposed that ROS1, DML2 and DML3 protect protein-coding genes from accumulating deleterious methylation as a consequence of their close proximity to transposons or repeated sequences.

In addition to DNA methylation, histone modifications are well-characterized epigenetic marks. The roles of individual histone modifications are generally conserved between plants and animals. There are eight types of histone modifications indexed (KOUZARIDES, Cell, 128, 693-705, 2007). These different types of modification can either activate or repress transcription but also affect other functions of the genome such as recombination or DNA repair. The mechanisms by which histones are modified at specific DNA target sites are for the moment unknown. One mechanism could involve recruitment of transcription factors; indeed, several plant transcription factors involved in development or stress response are known to interact with histone-modifying complexes. Once a histone is modified, it can serve as a platform to recruit protein complexes (e.g; transcription factors) that further modify neighbour histones, DNA methylation and/or transcription. As a result, histone methylation and DNA methylation are directly linked. Supporting this conclusion, mutations in the major H3K9 histone methyltransferase KRYPTONITE (KYP) decrease DNA methylation whereas mutations in the IBM1 H3K9 demethylase increase DNA methylation (SAZE & KAKUTANI, Embo J, 26, 3641-52, 2007).

In Arabidopsis, the pattern of DNA methylation is well characterised at the seedling state. Indeed, two recent studies have analysed the methylation pattern in the entire genome by hybridizing tiling arrays (ZHANG et al., Cell, 126, 1189-201, 2006; ZILBERMAN et al., Nat Genet, 39, 61-9, 2007) and observed that about 20% of the Arabidopsis genome was methylated. As expected, high levels of methylation were observed along the entire sequence length in regions with a high density of repetitive sequences, such as centromeric or knob regions. More surprising, approximately one third of genes contained patches of cytosine methylation in their coding and transcribed regions whereas far fewer genes (5%) presented this modification in their promoter regions. The methylated cytosines were mainly distributed within the body of the gene, suggesting that these epigenetic marks could inhibit cryptic transcriptional initiation outside of gene promoters. The comparison of the methylation map with a compilation of microarray expression data (SCHMID et al., Nat Genet, 37, 501-6, 2005) showed that core methylated genes are more frequently expressed at high levels and with low tissue-specificity, whereas promoter-methylated genes are expressed at lower levels but in a more tissue-specific manner (ZHANG et al., Cell, 126, 1189-201, 2006). By comparing *Arabidopsis* Col and Ler ecotypes, (VAUGHN et al., PLoS Biol, 5, e174, 2007) showed that methylation of repeated sequences is mostly stable while genic methylation is highly divergent. In general, gene methylation is unstable, highly polymorphic among individuals and populations and does not have a strong impact on gene expression. Consequently, in the *met1-3* background, only 1% of annotated genes were up-regulated and most of these were pseudogenes clustered in pericentromeric heterochromatic regions. Nevertheless, the continual maintenance of CG-methylation patterns has been shown recently to be a key factor in controlling transgenerational inheritance of epigenetic information including non-CG and histone methylation. In other words, altering the CG methylation process leads to severe alterations in non-CG and histone methylation. Therefore, methylation at CG sites not only functions to maintain stable silencing of TEs and pseudogenes, but also works as a "chef d'orchestre" of epigenetic marks. In contrast to the situation in the *met1* mutant, a large fraction (69%) of the genes up-regulated in the triple *drm1 drm2 cmt3* mutant are distributed throughout euchromatin. Because DRM1, DRM2 and CMT3 are mainly responsible for non-CG methylation, these results provide evidence that this type of methylation is important for regulating gene expression on a genome-wide scale.

It is reasonable to hypothesize that many traits are under epigenetic controls (MAKAREVITCH et al., Genetics, 177, 749-60, 2007), and that changes in DNA methylation or histone modification account for the plasticity of plant genome responses to developmental and environmental cues (CHEN & TIAN, Biochim Biophys Acta, 1769, 295-307, 2007; BENHAMED et al., Plant Cell, 18, 2893-903, 2006). However, only a few examples of stable epimutations have been documented in plants. A symmetric flower variant of *Linaria vulgaris* was described more than 250 years ago by Linnaeus. In this variant, the *Lcyc* gene is extensively methylated and transcriptionally silent. Occasionally, the variant reverts phenotypically during somatic development, correlating with demethylation of *Lcyc* and restoration of transcription (CUBAS et al., Nature, 401, 157-61, 1999). A second example is the tomato *colorless non-ripening* epimutant. In this variant, the promoter of an SBP-box gene is methylated, and this methylation is stably maintained over generations (MANNING et al., Nat Genet, 38, 948-52, 2006). In another example, the melon g epiallele results from the insertion of a transposable element 0.7 kb upstream of the G gene, causing transcriptional silencing and extensive methylation (MARTIN et al., Nature, 461, 1135-8, 2009). Two other examples, in Arabidopsis, illustrate that epimutants can result from either the gain or the loss of DNA methylation. The *sup* epimutant exhibits hypermethylation in the *SUP* gene, causing an increased number of stamens (JACOBSEN & MEYEROWITZ, Science, 277, 1100-3, 1997), while *fwa* epimutants carry a hypomethylated *FWA* gene and flower late (SOPPE et al., Mol Cell, 6, 791-802, 2000). Importantly, *FWA* normally is methylated in wild-type plants because of the presence of a direct repeat of a SINE element in its promoter (KINOSHITA et al., Plant J, 49, 38-45, 2007). Several other epimutations are associated with the presence of repeats. In certain Arabidopsis accessions, the tryptophan-biosynthetic *PAI1* gene is part of an inverted duplication, leading to trans-silencing of the unlinked *PAI2* gene, whereas in accessions carrying only one copy of the *PAI1* gene *PAI2* is expressed (BENDER & FINK, Cell, 83, 725-34, 1995). Other examples of silencing *in trans* include the maize pigmentation genes *Pl, B* and *R*. Increasing the copy number of repeated sequences in these gene promoters leads to epigenetic silencing in *cis,* silencing of allelic or ectopic copies of the wild-type gene *in trans* and creation of epialleles that themselves can cause silencing in trans, a phenomenon referred to as paramutation (CHANDLER, Cell, 128, 641-5, 2007).

Epimutations can arise naturally as a result of environmental stresses, or they can be induced artificially as a consequence of mutagenic laboratory treatments. Epimutations in the Arabidopsis *SUP* and *FWA* genes also arise at high frequency in *ddml* (DECREASE IN DNA METHYLATION 1) and *met1* mutants, which are relaxed in global DNA methylation (JACOBSEN et al., Curr Biol, 10, 179-86, 2000). Other examples of epimutations that arose in *ddm1* mutants are *bal* and *bsn* (STOKES & RICHARDS, Proc Natl Acad Sci U S A, 99, 7792-6, 2002; SAZE & KAKUTANI, Embo J, 26, 3641-52, 2007). Epimutations induced in *ddml* belong to two classes: i) recessive epimutations, such as *sup* and *bns,* which are associated with hypermethylation and transcriptional silencing of genes normally hypomethylated and active, and ii) semi-dominant epimutations, such as *fwa* and *bal,* which are associated with hypomethylation and transcriptional activation of genes normally silenced by DNA methylation. *ddm1* mutants exhibit a global decrease in DNA methylation, which is directly responsible for the hypomethylation observed at loci like *FWA* and *BAL* (VONGS et al., Science, 260, 1926-8, 1993). Nevertheless, the remaining methylation is often redistributed, leading sometimes to misplaced methylation such as that observed in the hypermethylated *sup* and *bns* epimutants (JACOBSEN et al., Curr Biol, 10, 179-86, 2000; SAZE & KAKUTANI, Embo J, 26, 3641-52, 2007). Epimutations like *bal, bns, fwa* and *sup* are stably inherited, even after a wild-type copy of *DDM1* is reintroduced by crossing to wild-type plants, indicating that their stability is independent from the event that induced them. The new epigenetic states at these loci are now stable and the plant does not have a memory of their ancient epigenetic state in these cases. However, a large number of DNA hypomethylation events induced in *ddm1* mutants revert to wild-type when a wild-type copy of *DDM1* is reintroduced. Remethylation at these loci correlates with the presence of 24-nucleotide siRNAs that guide DNA methylation at the right place (TEIXEIRA et al., Science, 323, 1600-4, 2009).

In plants, small RNAs (siRNAs and miRNAs) are processed from long double-stranded RNA (dsRNA) by DICER-LIKE (DCL) proteins, which belong to the RNaseIII class IV family of proteins.

RNaseIII enzymes (EC 3.1.26.3) are dsRNA-specific endonucleases found in bacteria and eukaryotes. All members of the RNaseIII family contain a characteristic RNaseIII domain (PROSITE PDOC00448), which has a highly conserved stretch of nine amino acid residues known as the RNaseIII signature motif (PROSITE PS00517).

RNaseIII proteins vary widely in length, from 200 to 2000 amino acids and have been subdivided into four classes based on domain composition.

Class I proteins are the simplest and the smallest, containing a single RNaseIII domain and a dsRNA binding domain (DRB; Prosite PDOC50137); the bacterial and bacteriophage RNaseIII belong to this class.

Class II proteins also comprise a single RNaseIII domain and a DRB domain; they further comprise a highly variable N-terminal domain extension and includes the *S. cerevisiae* Rnt1 and *S. pombe* Pac1 proteins. Both of these yeast proteins are longer than bacterial RNaseIII and contain an additional 100 amino acid fragment at the N-terminus.

Class III proteins, including Drosha proteins, have a DRB and two RNaseIII domains.

Class IV proteins, also referred to as Dicer, are the largest and contain a RNA helicase domain (Prosite PDOC51192), a Piwi/Argonaute/Zwille (PAZ; PROSITE PDOC50821) domain, two RNaseIII domains and one or two DRB domains.

Unless otherwise specified, the protein domains and motifs mentionned herein are defined by the accession number of their documentation entry in the PROSITE database (SIGRIST et al., Nucleic Acids Res. 38(Database issue)161-6, 2010).

So far, only DROSHA/DICER in animals and DCL in plants have been shown to produce small RNAs. After production in a dsRNA form, one strand of the small RNA duplex is loaded onto ARGONAUTE proteins, which execute gene silencing activity (VAUCHERET, Genes Dev, 20, 759-71, 2006).

In the plant model species Arabidopsis, DCL1 produces microRNA (miRNA) from imperfectly double-stranded stem-loop RNA precursors transcribed from non-protein coding MIR genes. miRNAs are involved in the post-transcriptional control of a variety of target genes including many developmental genes.

In contrast, siRNAs derive from dsRNA precursors, which originate from either convergent transcription of neighboring loci, inverted repeats, or from the action of RNA-dependent RNA polymerases (RDR) on precursor single-stranded RNAs. Several classes of siRNA are produced by different DCL. DCL4 produces 21-nt tasiRNAs from non-protein coding TAS RNA after they are converted into dsRNA by RDR6. tasiRNAs are loaded onto AGO1 to guide the cleavage of complementary mRNA. DCL3 produces 24-nt siRNAs from transposons and repeats RNA after they are converted into dsRNA by PolIV and RDR2. These 24-nt siRNAs associate with AGO4, which recruits PolV and DRD1, leading to transcriptional silencing through histone modification, DNA methylation and chromatin remodelling.

Despite their role in instructing DNA methylation, PollIV-RDR2-DCL3-dependent siRNAs appear dispensable for plants on the short term. Indeed, *polIV*, *rdr2* and *dcl3* mutants that lack 24-nt siRNAs do not exhibit obvious developmental phenotypes, suggesting that, once established, most epigenetic marks are maintained without further requirements for siRNAs.

The inventors hypothesized that ectopic expression of small RNA (up to 30 nt) s could modify the genome, and that such modification could be inherited after eliminating the siRNA trigger. To achieve ectopic expression of small RNAs, they decided to over-express RNaseIII enzymes referred to as RNASE THREE-LIKE (RTL), which do not belong to the DCL family.

The Arabidopsis genome contains five RTL genes. RTL1 (TAIR: AT4G15417; NCBI-GI: 240255863; UniProt: Q3EA18), RTL2 (TAIR: AT3G20420; NCBI-GI: 18402610; UniProt: Q9LTQ0 also represented herein as SEQ ID NO: 1), RTL3 (TAIR: AT5G45150; NCBI-GI: 15242329; UniProt: Q9FKF0), RTL4 (TAIR: AT1G24450; NCBI-GI: 15221749; UniProt: Q9FYL8), and RTL5 (TAIR: AT4G37510; NCBI-GI: 15235580; UniProt: Q9SZV0). In contrast with the DCL genes, the function of these RTL genes remains elusive, and it was not known until now if they had anything to do with small RNAs.

RTL1 mRNA has only been detected in roots where it is expressed at low levels. By contrast, RTL2 mRNA accumulates ubiquitously but at low levels, whereas RTL3 mRNA has never been detected. Like RTL2, RTL4 and RTL5 are more broadly expressed. To date, only rtl2 and rtl4 mutants have been described in Arabidopsis. rtl2 mutants are defective in the cleavage of the 3'external transcribed spacer (ETS) of the pre-rRNA (COMELLA et al., Nucleic Acids Res, 36, 1163-75, 2008). However, this function does not appear essential for plant development because rtl2 mutants do not exhibit any obvious developmental defects. By contrast, rtl4 (also referred to as nfd2) homozygous mutants could not be obtained because rtl4 mutations impact both male and female gametophytes (PORTEREIKO et al., Plant Physiol, 141, 957-65, 2006). Only rtl4/RTL4 heterozygous plants can be propagated, which, owing to the poor viability of rtl4 gametes, produced ca. 95% RTL4/RTL4 plants and only 5% rtl4/RTL4 plants after self-fertilization. The molecular function of RTL4 is still unknown.

AtRTL1 is 289 amino acids long and comprises one RNaseIII domain and no conserved RNA-binding domain; AtRTL2 is 391 amino acids long and comprises a single RNaseIII domain and a DRB; AtRTL3 is 957 amino acids long and comprises two RNaseIII domains and three DRBs. In contrast with the DCL proteins, these RTL proteins do not comprise multifunctional domains such as RNA helicase and PAZ domains (COMELLA et al., Nucleic Acids Res, 36, 1163-75, 2008). Also, RTL genes normally are expressed at very low levels in wild-type plants whereas DCL genes are more highly expressed.

The inventors have found that AtRTL2 has the capacity to produce small RNAs, including in particular siRNAs, and that introduction in a plant of a transgene expressing this enzyme leads to a variety of genetic and epigenetic changes that can be stably inherited, even after the RTL2 transgene has been removed.

The invention therefore provides a method for producing plants having one or more genetic mutation(s) and/or one or more heritable epimutation(s), wherein said method comprises:
a) producing transgenic plants by transforming said plants with a DNA construct comprising a sequence coding a RNAse III protein under transcriptional control of an appropriate promoter, said RNAse III protein having one or more RNaseIII domain(s) and one or more DRB(s);
b) selecting among the transgenic plants of step a) expressing said RNaseIII protein a plant having at least one phenotypic change (which is indicative of the presence of a genetic mutation or of an epimutation);
c) crossing said transgenic plant with a wild-type plant (i.e a plant which does not comprise the transgene encoding the RNAse III protein);
d) selecting among the progeny of step c) a plant having lost the transgene and having kept the phenotypic change of step b).

The presence of phenotypic changes in the plants of steps b) and c) can be determined by classical methods well known in themselves, such as visual inspection, screening under selective conditions (for instance salt stress, drought stress, etc), and the like.

RNAse III proteins which are particularly suitable for use in the method of the invention are those able to induce when transiently expressed in *Nicotiana benthamiana* leaves together with an inverted repeat construct which produces dsRNA, the accumulation of small RNAs derived from said dsRNA, said small RNAs comprising in particular 24-nt siRNAs.

According to a preferred embodiment of the invention, the RNAse III protein has a single RNaseIII domain, and one DRB. According to a particular embodiment, it has no RNA helicase and PAZ domains.

Preferably, said RNAse III protein has the following characteristics:
- its RNaseIII domain has at least 74% sequence similarity with SEQ ID NO: 2 (LKEA ITHTSCTDFP SYERLEFIGD SAIGLAISNY LYLTYPSLEP HDLSLLRAAN VSTEKLARVS LNHGLYSFLR RNAPSLDEKV KEFSEAVGKE DDLSVSYGGL VKAPKVLADL FESLAGAVYV DVNFDLQRLW), which represents the RNaseIII domain of the AtRTL2 protein of *Arabidopsis thaliana*; and
- its DRB domain has at least 56% similarity with SEQ ID NO: 3 (VIFRGLLEPI VTLDDLQKQP QPVSMLFKLC HKHKKRIDIK NWKDGNVSIA VIYLDDELLA SGRAENKDIA RLIAAKEALR KLSEVFPVEM VIDEDSVEIQ LTHAKTKLNE ICLKKKWPKP IYSVEEDRSS VQGKRFVCSA KIKITEEKTL YMKGDEQSKI KKAESSSAYH MIRALRKSHY L) which represents the DRB domain of the AtRTL2 protein of *Arabidopsis thaliana.*

The sequence identity and similarity values indicated herein are calculated using the Tblastn program (available at at the NCBI website: http://www.ncbi.nlm.nih.gov/blast) under default parameters. Similarity calculations are performed using the scoring matrix BLOSUM62.

Besides the AtRTL2 protein of *Arabidopsis thaliana,* non-limitative examples of RNAse III proteins that fulfil the above-defined criteria include those listed in Table 1 below:

**Table 1**

| **RNAseIII Domain** | | | | | |
|---|---|---|---|---|---|
| Species | Nature of the sequence | Database reference of the sequence | Identity with SEQ ID NO: 2 | Similarity with SEQ ID NO: 2 | Score |
| *Arabidopsis lyrata* | hypothetical protein | GENE ID: 9319311 ARALYDRAFT_479566 | 126/134 (94%), | 131/134 (97%), | 263 |
| *Ricinus communis* | putative mRNA | GENE ID: 8278929 RCOM_0752600 | 91/134 (67%), | 118/134 (88%), | 195 |
| *Populus trichocarpa* | predicted protein, | GENE ID: 7483612 POPTRDRAFT_660602 mRNA | 89/135 (65%), | 116/135 (85%), | 190 |
| *Vitis vinifera* | hypothetical protein | GENE ID: 100243580 LOC100243580 | 87/134 (64%), | 111/134 (82%), | 178 |
| *Solanum lycopersicum* | cDNA | dbj\|AK321055.1\| | 83/134 (61%), | 112/134 (83%), | 175 |
| *Lotus japonicus* | genomic DNA | dbj\|AP010537.1\| | 86/134 (64%), | 101/134 (75%), | 167 |
| *Phyllostachys edulis* | cDNA | emb\|FP097839.1\| | 87/139 (62%), | 106/139 (76%), | 164 |
| *Oryza sativa* | cDNA | emb\|CT836411.1\| | 88/139 (63%), | 106/139 (76%), | 163 |
| *Zea mays* | cDNA | GENE ID: 100272891 LOC100272891 | 82/139 (58%), | 104/139 (74%), | 156 |
| *Sorghum bicolor* | hypothetical protein | GENE ID: 8061288 SORBIDRAFT_09g013160 | 81/139 (58%), | 103/139 (74%), | 152 |
| *M.truncatula* | DNA sequence | emb\|CR936364.7\| | 74/135 (54%), | 105/135 (77%), | 149 |
| | | | | | |

| **DRB Domain** | | | | | |
|---|---|---|---|---|---|
| Species | Nature of the sequence | Database reference of the sequence | Identity with SEQ ID NO: 3 | Similarity with SEQ ID NO: 3 | |
| *Arabidopsis lyrata* | hypothetical protein | GENE ID: 9319311 ARALYDRAFT 479566 | 170/181 (93%), | 170/181 (93%), | 351 |
| *Ricinus communis* | putative mRNA | GENE ID: 8278929 RCOM_0752600 | 93/186 (50%), | 134/186 (72%), | 186 |
| *Vitis vinifera* | hypothetical protein | GENE ID: 100243580 LOC100243580 | 87/185 (47%), | 127/185 (68%), | 162 |
| *Solanum lycopersicum* | cDNA | dbj\|AK321055.1\| | 70/176 (39%), | 116/176 (65%), | 142 |
| *Populus trichocarpa* | predicted protein, | GENE ID: 7483612 POPTRDRAFT_660602 mRNA | 70/128 (54%), | 90/128 (70%), | 137 |
| *M. truncatula* | DNA sequence | emb\|CR936364.7\| | 56/125 (44%), | 87/125 (69%), | 123 |
| *Phyllostachys edulis* | cDNA | emb\|FP097839.1\| | 66/181 (36%), | 102/181 (56%), | 115 |
| *Sorghum bicolor* | hypothetical protein | GENE ID: 8061288 SORBIDRAFT 09q013160 | 61/182 (33%), | 104/182 (57%), | 111 |
| *Zea mays* | cDNA | GENE ID: 100272891 LOC100272891 | 60/181 (33%), | 103/181 (56%), | 107 |
| *Lotus japonicus* | genomic DNA | dbj\|AP010537.1\| | 46/86 (53%), | 66/86 (76%), | 100 |
| *Oryza sativa* | cDNA | emb\|CT836411.1\| | 56/176 (31%), | 99/176 (56%), | 98,6 |

The invention also comprises transformed plant cells or plants containing a DNA construct comprising a sequence encoding a RNAse III protein as defined above, under transcriptional control of an appropriate promoter. The expression of said RNAse III protein induces genetic mutations as well as epimutations in said plants or plant cells.

The invention also includes recombinant DNA constructs for expressing a RNAse III protein as defined above, in a host-cell, or a host organism, in particular a plant cell or a plant. These DNA constructs can be obtained and introduced into said host cell or organism by well-known techniques of recombinant DNA and genetic engineering.

Recombinant DNA constructs of the invention comprise a polynucleotide encoding said RNAse III protein, under the control of an appropriate promoter.

Said promoter can be any promoter functional in a plant cell. The choice of the more appropriate promoter may depend in particular on the chosen host plant, on the organ(s) or tissue(s) targeted for expression, and on the type of expression (i.e. constitutive or inducible) that one wishes to obtain.

A large choice of promoters suitable for expression of heterologous genes in plants is available in the art. They can be obtained for instance from plants, plant viruses, or bacteria such as *Agrobacterium.* They include constitutive promoters, i.e. promoters which are active in most tissues and cells and under most environmental conditions, tissue or cell specific promoters which are active only or mainly in certain tissues or certain cell types, and inducible promoters that are activated by physical or chemical stimuli, such as those resulting from water deficit.

Non-limitative examples of constitutive promoters that are commonly used in plant cells are the cauliflower mosaic virus (CaMV) 35S promoter, the Nos promoter, the rubisco promoter, the Cassava vein Mosaic Virus (CsVMV) promoter, the rice actin promoter, followed by the rice actin intron (RAP-RAI) contained in the plasmid pActl-F4 (MCELROY et al., Molecular and General Genetics, 231(1), 150-160, 1991)

Non-limitative examples of organ or tissue specific promoters which that can be used in the present invention include for instance:
- Seed-specific promoters (VIGEOLAS et al., Plant Biotechnol J, 5, 431-41, 2007);
- Leaf-specific promoters (PETERSON & OLIVER, Plant Physiol Biochem, 44, 885-92, 2006);
- Stem-specific promoters (SAHA et al., Planta, 226, 429-42, 2007);
- Root-specific promoters (SIVANANDAN et al., Biochim Biophys Acta, 1731, 202-8, 2005);
- Flower-specific promoters (MAIZEL & WEIGEL, Plant J, 38, 164-71, 2004);
- Pollen-specific promoters (YANG et al., Genetika, 46, 458-63), in particular constitutive promoters (DAS et al., Transgenic Res);
- Embryo-specific promoters (GUO et al., Planta, 231, 293-303);
- Promoters expressed in non-differentiated or proliferating tissues (US 6 031 151);
- Meristem-specific promoters (MAIZEL & WEIGEL, Plant J, 38, 164-71, 2004);
   Inducible promoters that can be used in the present invention include for instance: Ethanol-inducible promoters (ALVAREZ et al., Plant Mol Biol, 68, 61-79, 2008);
- Drought-inducible promoters (DOCZI et al., Plant Physiol Biochem, 43, 269-76, 2005);
- Cold-inducible promoters (KHODAKOVSKAYA et al., Planta, 223, 1090-100, 2006).
- The expression cassette generally also includes a transcriptional terminator, such as the 35S transcriptional terminator. They may also include other regulatory sequences, such as transcription enhancer sequences or introns (for example the FAD2 intron described in WO 2006/003186, or the actin intron (MCELROY et al., 1991 cited above). Among the terminators which can be used in the constructs of the invention, mention may be made in particular of the 3' end of the Agrobacterium tumefaciens nopaline synthase gene (DEPICKER et al., J Mol Appl Genet. 1(4):361-370, 1982). Mention may also be made of the 35S polyA terminator of the cauliflower mosaic virus (CaMV), described by FRANCK et al. (Cell. 21(1):285-94, 1980).

The invention also includes recombinant vectors containing a recombinant DNA construct of the invention. Classically, said recombinant vectors also include one or more marker genes, which allow for selection of transformed hosts. As non-limitative examples of marker genes, mention may be made of genes which confers resistance to an antibiotic, for example to hygromycin (HERRERA-ESTRELLA et al., EMBO J. 2(6): 987-995 1983) or resistance to an herbicide such as the sulfonamide asulam (PCT WO 98/49316).

The selection of suitable vectors and the methods for inserting DNA constructs therein are well known to persons of ordinary skill in the art. The choice of the vector depends on the intended host and on the intended method of transformation of said host. A variety of methods for genetic transformation of plant cells or plants are available in the art for most of plant species, dicotyledons or monocotyledons. By way of non-limitative examples, one can mention virus mediated transformation, transformation by microinjection, by electroporation, microprojectile mediated transformation, *Agrobacterium* mediated transformation, and the like.

The method of the invention can be used in a broad variety of plants including dicotyledons as well as monocotyledons, and in particular plants of agronomical interest, as crop plants, as fruit, vegetables or ornamental plants. In particular it can be used in Brassicales, in particular those of the *Brassicaceae* family.

The present invention will be further illustrated by the additional description which follows, which refers to examples illustrating the effects of over-expression of Arabidopsis RTL genes and the generation of genetic mutations and epimutations by over-expression of RTL2. It should be understood however that these examples are given only by way of illustration of the invention and do not constitute in any way a limitation thereof.

### EXAMPLE 1: EFFECT OF OVER-EXPRESSION OF ARABIDOPSIS RTL GENES ON SMALL RNA DERIVED FROM AN ARTIFICIAL DSRNA

To gain insight in the function of the five RTL genes of *Arabidopsis thaliana,* agrobacterium-mediated infiltration in *Nicotiana benthamiana* leaves was used to introduce constructs expressing each RTL under the control of the strong constitutive cauliflower mosaic virus (CaMV) 35S promoter, together with a GUS inverted repeat construct (35S-IRGUS), which produces GUS dsRNA.

### 35S-RTL constructs

Sequences encoding RTL1, RTL2 and RTL3 were amplified from *Arabidopsis thaliana* genomic DNA, and sequences encoding RTL4 and RTL5 were cloned from *Arabidopsis thaliana* cDNAs, using the primers listed in Table 2 below.

**Table 2**

| Primers | Sequence (SEQ ID NO: #) | Restriction enzymes |
|---|---|---|
| RTL1-F | cgacctgcagATGGGATCGCAACTCTCAA (4) | Pst1 |
| RTL1-R | cggcgaattcCTAAAGGTCTCCGAAAAATT (5) | EcoR1 |
| RTL2-F | cgcgggatccCTCTCTACCATGGATCACTC (6) | BamH1 |
| RTL2-R | cgcggaattcCTGAACTCTGTCCATAACGG (7) | EcoR1 |
| RTL3-F | cggctgcagATGAATTCAGTAGAAGCAGTAG (8) | Pst1 |
| RTL3-R | cggcggatccTCATAGTCTTCTCCTCCTCCTTT (9) | BamH1 |
| RTL4-F | cggcggatccATGGCGACTCTTCGTTTCACTC (10) | BamH1 |
| RTL4-R | cggcgaattcTTACAACATAGAGACTAGTCTTC (11) | EcoR1 |
| RTL5-F | cggcggtcgacATGGAGCTTTGTTCTTCATCTC (12) | SalI |
| RTL5-R | cggcggaattcTCAGACAGCTTTAGGTTGGAT (13) | EcoRI |

The PCR amplification was performed using the Phusion® High-Fidelity DNA polymerase as suggested by the supplier (Finnzymes).

PCR products were digested by the appropriate restriction enzymes and ligated between the 35S promoter and the 35S terminator of the pLBR19 vector (GUERINEAU et al., Plant Mol Biol, 18, 815-8, 1992), then transferred to the pGreenII0129 vector (HELLENS et al., Plant Mol Biol, 42, 819-32, 2000).

### 35S-IRGUS construct and 35S-GFP construct

35S-IRGUS is described in WATERHOUSE et al. (Proc Natl Acad Sci U S A, 95, 13959-64, 1998).

35S-GFP is described for instance in LEFFEL et al., (Biotechniques, 5 , 912-918, 1997).

### Plant transformation

Constructs were introduced into *Agrobacterium* strain C58-pmp90 (KONCZ & Schell, Mol Gen Genet, 204, 383-396, 1986), by electroporation, as described by WISE et al (Methods Mol Biol, 343, 43-53, 2006).

*Nicotiana benthamiana* leaves were transformed by infiltration as described by SCHÖB et al. (Mol Gen Genet, 256, 581-5, 1997).

As a control, the 35S-IRGUS construct was agroinfiltrated with the 35S-GFP construct.

### Quantification of of siRNAs

Levels of siRNAs were determined as described in PALL et al (Nucleic Acids Res, 35, e60, 2007).

### Results:

In control 35S-IRGUS/35S-GFP infiltrated leaves, the GUS dsRNA derived from 35S-IRGUS is processed into 21-nt siRNA by the endogenous DCL4 activity.

In 35S-IRGUS/35S-RTL3, 35S-IRGUS/35S-RTL4 and 35S-IRGUS/35S-RTL5 infiltrated leaves, GUS siRNA level was similar to that of control 35S-IRGUS/35S-GFP infiltrated leaves, suggesting that RTL3, RTL4 and RTL5 neither produce siRNA nor interfere with siRNA accumulation.

In contrast, 35S-IRGUS/35S-RTL1 infiltrated leaves lacked GUS siRNA, indicating that RTL1 impairs siRNA accumulation, either by sequestrating GUS dsRNA, or degrading GUS dsRNA into undetectable fragments, or degrading GUS siRNA produced by the endogenous *Nicotiana benthamiana* DCL4 activity.

Lastly, 35S-IRGUS/35S-RTL2 infiltrated leaves exhibited GUS 21-nt siRNA to a level similar to that of control 35S-IRGUS/35S-GFP infiltrated leaves, but, in addition, accumulated GUS 24-nt siRNA, suggesting that RTL2 processes dsRNA into 24-nt siRNA.

### EXAMPLE 2: THE RNASEIII CATALYTIC DOMAIN OF RTL1, RTL2 AND RTL5 IS ESSENTIAL FOR THEIR ACTIVITY

To determine if the RNaseIII catalytic domain of RTL proteins is required for their activity, we mutagenized two conserved amino acids of the RNaseIII catalytic domain as defined by the functional analysis of *Aquifex aeolicus* RNaseIII (BLASZCZYK et al., Structure, 9, 1225-36, 2001).

### Site-directed mutagenesis

Each 35S::RTL construct was mutagenized using QuikChange® XL Site-Directed Mutagenesis Kit (Stratagen) with the Pfu Turbo® DNA polymerase (Stratagen).

The list of primers used for mutagenesis is indicated in Table 3 below.

**Table 3**

| Primers | Sequence (SEQ ID NO: #) | Mutation |
|---|---|---|
| | | |
| RTL1 m-F | GAATCGTACGcACTCCTAGAACTTTTAGGAGcTTCGATCC (14) | GAA (E64)->GcA (A64); GAT (D71)->GcT (A71) |
| RTL1 m-R | GGATCGAAgCTCCTAAAAGTTCTAGGAGTgCGTACGATTC (15) | |
| RTL2m-F | CCTTCTTACGcGCGGCTAGAGTTCATAGGCGccAGTGCTATTGG (16) | GAG (E93)->GcG (A93); GAT (D100)->Gcc (A100) |
| RTL2m-R | CCAATAGCACTggCGCCTATGAACTCTAGCCGCgCGTAAGAAGG (17) | |
| RTL3m-F | CCTTTATTCGcCCGGCTTGAGTTCTTCGGCGcCTCTATTCTTGAGG (18) | GAC (D33)->GcC (A37); GAC (D40)->GcC (A40) |
| RTL3m-R | CCTCAAGAATAGAGgCGCCGAAGAACTCAAGCCGGgCGAATAAAGG (19) | |
| RTL4m-F | GAGAACAACgcAGCCTTGAGTATCTTTGGAgCCCATATCATTG (20) | AAA (K80)->gcA (A80); ACC (T87)->gCC (A87) |
| RTL4m-R | CAATGATATGGGcTCCAAAGATACTCAAGGCTgcGTTGTTCTC (21) | |
| RTL5m-F | GGTTTTGTTTTGcGCGGCTGGAATATGTAGGAgcGAAGATACAGG (22) | GAG (E422)-> GcG (A422); CAG (Q429)->gcG (A429) |
| RTL5m-R | CCTGTATCTTCgcTCCTACATATTCCAGCCGCgCAAAACAAAACC (23) | |

RNaseIII-defective RTL are hereafter referred to as RTLm.

RTL1m has a E > A substitution at position 64, and a D > A substitution at position 71 of the peptide sequence of RTL1; RTL2m has a E > A substitution at position 93, and a D > A substitution at position 100 of the peptide sequence of RTL2; RTL3m has a D > A substitution at position 33, and a D > A substitution at position 40 of the peptide sequence of RTL3; RTL4m has a K > A substitution at position 80, and a T > A substitution at position 87 of the peptide sequence of RTL4; RTL5m has a E > A substitution at position 422, and a Q > A substitution at position 429 of the peptide sequence of RTL5.

We first tested if the RNaseIII catalytic domain is required for the effect of RTLs on GUS siRNA accumulation.

In 35S-IRGUS/35S-RTL3, 35S-IRGUS/35S-RTL3m, 35S-IRGUS/35S-RTL4, 35S-IRGUS/35S-RTL4m, 35S-IRGUS/35S-RTL5 and 35S-IRGUS/35S-RTL5m infiltrated leaves, GUS siRNA level was similar to that in control 35S-IRGUS/35S-GFP infiltrated leaves, confirming that RTL3, RTL4 and RTL5 neither produces siRNA nor interferes with siRNA accumulation.

In 35S-IRGUS/35S-RTL1m infiltrated leaves, GUS siRNA levels were similar to that of control 35S-IRGUS/35S-GFP infiltrated leaves, indicating that a functional RNaseIII catalytic domain is required for 35S-RTL1 to interfere with siRNA accumulation.

In 35S-IRGUS/35S-RTL2m infiltrated leaves, the accumulation of GUS 24-nt siRNA was abolished while GUS 21-nt siRNA level was similar to that of control 35S-IRGUS/35S-GFP infiltrated leaves, indicating that a functional RNaseIII catalytic domain is required for 35S-RTL2 to produce 24-nt siRNA.

Because RTL5 is essential for plant development, we also tested if the RNaseIII catalytic domain of RTL5 is required for its activity. A 35S-RTL5 construct, but not a 35S-RTL5m construct, was able to complement the rtl5 mutant embryolethality, indicating that RTL5 also acts through its RNaseIII catalytic domain.

### EXAMPLE 3: DEVELOPMENTAL DEFECTS RESULTING FROM OVER-EXPRESSION OF ARABIDOPSIS RTL GENES

To gain insight on the function of the five Arabidopsis RTL genes, each 35S-RTL construct was introduced into wildtype Arabidopsis by agrobacterium-mediated floral dipping method (CLOUGH & BENT, Plant J, 16, 735-43, 1998).

Transformants were selected on 1/2×MS medium supplemented with Gamborg vitamins (Sigma), 10 g/l saccharose and hygromycin 30mg/l.

35S-RTL3, 35S-RTL4 and 35S-RTL5 primary transformants (T1 generation) did not exhibit obvious developmental defects.

By contrast, 44 out of 46 35S-RTL1 primary transformants exhibited a chlorotic phenotype and were self-sterile. The remaining two transformants, which looked like wildtype plants, did not express the 35S-RTL1 transgene, suggesting that expression of the 35S-RTL1 construct is responsible for the chlorotic/sterility phenotype.

To test this hypothesis, the 35S-RTL1m construct was introduced into wildtype Arabidopsis. All 27 35S-RTL1m transformants looked like wildtype plants, although the 35S-RTL1m transgene was expressed, indicating that the RNaseIII activity of the 35S-RTL1 construct is responsible for the chlorotic/sterility phenotype.

Thirteen out of 68 35S-RTL2 primary transformants exhibited a variety of developmental defects, including chlorosis, dwarfness, bushyness, late flowering and sterility. Such a diversity of defects is not normally observed after transformation of Arabidopsis by agrobacterium, suggesting that expression of the 35S-RTL2 construct is responsible for this variety of developmental defects.

Moreover, 9 out of the 55 wildtype looking 35S-RTL2 primary transformants produced progeny plants (T2 generation) with developmental defects. In one case, the phenotype was observed in 25% of the progeny and could be attributed to the inactivation of an endogenous gene by the T-DNA carrying the 35S-RTL2 construct (this was confirmed by the fact that all plants with this phenotype were homozygous for the 35S-RTL2 transgene).

In the 8 remaining lines, developmental defects appeared at frequencies ranging between 1% and 12%, which are not compatible with the inactivation of an endogenous gene by the T-DNA carrying the 35S-RTL2 construct. Moreover, one phenotype (dwarf, chlorotic, late flowering) was observed in the progeny of 5 independent transformants, while another phenotype (dark green, downward curved leaves) was observed in the progeny of 2 independent transformants. Because T-DNA insertion occurs randomly within the genome, the possibility that these phenotypes are caused by the inactivation of endogenous genes by the T-DNA carrying the 35S-RTL2 transgene was ruled out.

### EXAMPLE 4: EPIMUTATIONS CAUSED BY OVER-EXPRESSION OF ARABIDOPSIS RTL2 CAN BE INHERITED INDEPENDENTLY OF THE 35S-RTL2 TRANSGENE.

The appearance of a variety of developmental defects among 35S-RTL2 transformants suggests that RTL over-expression could trigger genetic or epigenetic changes. Epigenetic changes are characterized by their heritability after elimination of the original trigger and their reversibility at frequencies higher than that of mutations. Two phenotypes that appeared in the T2 progeny of 35S-RTL2 transformants fit with this definition.

The first phenotype corresponds to small plant with downward curled leaves (hereafter referred to as "small/zip") derived from one 35S-RTL2 primary transformant. Self-fertilization of this plant yielded a variety of phenotypes, including wildtype looking plants, small plants with serrated leaves, "small/zip" plants, and dwarf and sterile plants. One "small/zip" plant lacked the 35S-RTL2 transgene, indicating that this the developmental defect can be inherited independent of the T-DNA carrying the 35S-RTL transgene. Further analyses were performed on the progeny of this transgene-free "small/zip" plant in order to analyze the heritability of this character in the absence of the 35S-RTL2 transgene.

Self-fertilization of this plant yield wildtype, "small/zip" and "dwarf/sterile" plants, suggesting that the transgene-free "small/zip" plant from which they derive did not carry the "serrated" character but carried the "small/zip" and the "dwarf/sterile" characters. Subsequent self-fertilization of "small/zip" plants again yield wildtype, "small/zip" and "dwarf/sterile" plants. Another round of self-fertilization of "small/zip" plants yield wildtype, "small/zip" and "dwarf/sterile" plants, suggesting that "small/zip" and "dwarf/sterile" phenotypes cannot be separated.

The simplest explanation is that both phenotypes result from a semi-dominant epimutation (or a group of genetically linked semi-dominant epimutations), and that "small/zip" are heterozygous whereas "dwarf/sterile" are homozygous.

Supporting this hypothesis, crosses between "small/zip" plants and wildtype plants yielded wildtype and "small/zip" plants F1 plants, as expected for a plant heterozygous for a semi-dominant character. However, the frequency of wildtype, "small/zip" and "dwarf/sterile" plants was not exactly that expected for a semi-dominant epimutation. Indeed, crosses between "small/zip" plants and wildtype plants yield ca. 88% wildtype and 12% "small/zip" plants F1 plants (instead of the expected 50% and 50%). Moreover, self-fertilization of "small/zip" plants yield 46% wildtype, 47% "small/zip" and 7% "dwarf/sterile" (instead of the expected 25%, 50% and 25%), suggesting that the epimutation (or the group of genetically linked semi-dominant epimutations) is unstable and often reverts to wildtype. Based on these segregations, the reversion frequency was estimated at 40%.

The second phenotype analyzed corresponded to plants with serrated leaves (hereafter referred to as "serrated") derived from another 35S-RTL2 primary transformant.

Self fertilization of "serrated" plants yield wildtype, "serrated" and "dwarF' plants (which produced a few seeds whereas the "dwarf/sterile" plants described above were totally sterile). Subsequent self-fertilization of "serrated" plants again yielded wildtype, "serrated" and "dwarf plants, whereas the very few seeds harvested on "dwarf" plants yielded mostly "dwarf" plants.

Therefore, we hypothesized that "serrated" and "dwarf" phenotypes both resulted from a semi-dominant epimutation (or a group of genetically linked semi-dominant epimutations), and that "serrated" plants were heterozygous whereas "dwarf" plants were homozygous.

However, the frequency of wildtype, "serrated" and "dwarf" phenotypes was not exactly that expected for a semi-dominant epimutation. Indeed, self-fertilization of "serrated" plants yielded 33% wildtype, 49% "serrated" and 18% "dwarf", while self-fertilization of "dwarf" plants yielded 0,5% wildtype, 10% "serrated" and 89,5% "dwarf", suggesting that the epimutation (or the group of genetically linked semi-dominant epimutations) is unstable and occasionally reverts to wildtype. Based on the segregation ratio, reversion frequency was estimated to be 7%. Moreover, somatic reversion from "dwarf" to "serrated" and from "serrated" to wildtype also was observed, which is characteristic of epimutations. Self-fertilization of revertant "serrated" plants yield wildtype, "serrated" and "dwarf" at the same frequency as a regular "serrated" plants, whereas revertant wildtype plants yielded 100% wildtype plants, indicating that the reversion had affected the germline. PCR screening did not identify "serrated" or "dwarf" plants that lacked the 35S-RTL2 transgene, suggesting either that these phenotypes, although unstable, require the constant presence of the 35S-RTL2 transgene, or that the epimutation(s) is (are) genetically linked to the locus carrying the 35S-RTL2 transgene.

### EXAMPLE 5: DELETIONS CAUSED BY OVER-EXPRESSION OF ARABIDOPSIS RTL2

One phenotype (dwarf plants with chlorotic leaves, which flowered late, hereafter referred to as "dwarf/chlorotic/late") was observed in T2 plants derived from five independent 35S-RTL2 primary transformants. Self-fertilization of these plants yielded 100% "dwarf/chlorotic/late" plants, indicating that this phenotype is stably transmitted to the progeny.

Crosses between "dwarf/chlorotic/late" plants and wildtype plants yield 100% wildtype looking F1 plants, indicating that the "dwarf/chlorotic/late" phenotype behaves as a recessive trait. Self-fertilization of these F1 plants yield 75% wildtype looking F2 plants and 25% "dwarf/chlorotic/late" F2 plants, indicating that this phenotype segregates as a monogenic character.

Some of the "dwarf/chlorotic/late" F2 lacked the 35S-RTL2 transgene, indicating that the maintenance of this phenotype does not require the constant presence of the 35S-RTL2 transgene. Self-fertilization of these transgene-free "dwarf/chlorotic/late" plants yielded 100% "dwarf/chlorotic/late" plants, indicating that this phenotype is stable in the absence of the 35S-RTL2 transgene. The stability of this trait was confirmed during two additional generations.

Lastly, all crosses between "dwarf/chlorotic/late" plants derived from the five independent 35S-RTL2 primary transformants yielded 100% "dwarf/chlorotic/late" plants, indicating that these five independent variants are allelic. Altogether, these data strongly suggest that the "dwarf/chlorotic/late" phenotype results from a recessive mutation or epimutation (or a group of genetically linked recessive mutations or epimutations) that has been induced independently in five 35S-RTL2 transformants.

Analysis of 200 "dwarf/chlorotic/late" F2 progeny of a cross between the original "dwarf/chlorotic/late" plants (in the Col ecotype) and wildtype plants from the Ler ecotype, allowed us to genetically map the "dwarf/chlorotic/late" phenotype between nucleotide position 10,428,000 and 13,920,000 on chromosome 5. Even after analyzing 200 F2 plants, the mapping interval remained uncharacteristically large (3,5 Mb), and increasing the number of F2 plants analyzed did not reduce this interval. Examination of this interval revealed that it contains the centromere of the chromosome, explaining why no cross-over could be observed in this region. Like every pericentromeric region, it is poor in protein-coding genes but rich in transposons, repeats and pseudogenes.

Transcriptomic analysis of "dwarf/chlorotic/late" plants derived from two independent 35S-RTL2 transformants revealed that eight protein-coding genes located in the middle of the interval are down-regulated whereas two protein-coding genes located at the edge of the interval are upregulated.

To explain the simultaneous down-regulation of eight adjacent genes, we hypothesized that the region carrying these eight genes could have been deleted. PCR analysis of the interval with primers located every 50 kbs revealed that a region of about 1000 kbs containing the eight down-regulated genes is absent in the "dwarf/chlorotic/late" plants, confirming our hypothesis.

## Claims

1. A method for producing plants having one or more genetic mutation(s) and/or one or more heritable epimutation(s), wherein said method comprises:
a) producing transgenic plants by transforming said plants with a DNA construct comprising a sequence coding a RNAse III protein under transcriptional control of an appropriate promoter, said RNAse III protein having one or more RNaseIII domain(s) and one or more dsRNA-binding domain(s)(DRB);
b) selecting among the transgenic plants of step a) expressing said RNaseIII protein a plant having at least one phenotypic change ;
c) crossing said transgenic plant with a wild-type plant (i.e a plant which does not comprise the transgene encoding the RNAse III protein);
d) selecting among the progeny of step c) a plant having lost the transgene and having kept the phenotypic change of step b).

2. The method of claim 1, wherein the RNAse III protein has a single RNaseIII domain, and one DRB.

3. The method of claim 2, wherein said RNAse III protein has the following characteristics:
- its RNaseIII domain has at least 74% sequence similarity with SEQ ID NO: 2;
and
- its DRB has at least 56% similarity with SEQ ID NO: 3.

4. A genetically transformed plant cell containing a DNA construct comprising a sequence encoding a RNAse III protein as defined in any of claims 1 to 3, under transcriptional control of an appropriate promoter.

5. A transgenic plant containing a DNA construct comprising a sequence encoding a RNAse III protein as defined in any of claims 1 to 3, under transcriptional control of an appropriate promoter.

6. A recombinant DNA construct containing a sequence encoding a RNAse III protein as defined in any of claims 1 to 3, under transcriptional control of an appropriate promoter.
